Europäisches Patentamt

European Patent Office

Office européen des brevets

(11) Veröffentlichungsnummer: **0 044 030**
**B1**

(12) **EUROPÄISCHE PATENTSCHRIFT**

(45) Veröffentlichungstag der Patentschrift:
03.10.84

(51) Int. Cl.³: **C 08 G 59/42,** C 09 D 5/40,
C 09 D 3/58

(21) Anmeldenummer: 81105293.5

(22) Anmeldetag: 08.07.81

(54) Verfahren zur Herstellung von Epoxidharz-Pulverlacken mit Härtern aus Monosalzen von aromatischen Polycarbonsäuren.

(30) Priorität: 12.07.80 DE 3026455

(43) Veröffentlichungstag der Anmeldung:
20.01.82 Patentblatt 82/3

(45) Bekanntmachung des Hinweises auf die Patenterteilung:
03.10.84 Patentblatt 84/40

(84) Benannte Vertragsstaaten:
BE CH DE FR GB IT LI LU NL SE

(56) Entgegenhaltungen:
DE - A - 1 545 187
DE - A - 2 630 011
FR - A - 2 229 747

(73) Patentinhaber: CHEMISCHE WERKE HÜLS AG,
Postfach 1320, D-4370 Marl 1 (DE)

(72) Erfinder: Gude, Fritz, Dr., Wilhelmstrasse 4,
D-4690 Herne 2 (DE)
Erfinder: Neubold, Kurt, Dr., Lindenstrasse 57,
D-4390 Gladbeck (DE)
Erfinder: Riemer, Heinz, Dr., Fritz-Reuter-Strasse 15,
D-4272 Kirchhellen (DE)
Erfinder: Dörmann, Günter, Händelstrasse 59,
D-4630 Bochum (DE)

(74) Vertreter: Steil, Hanna, Dipl.-Chem., RSP PATENTE -
PB 15 Postfach 1320, D-4370 Marl 1 (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Aus der DE-PS 2 324 696 ist bekannt, z.B. Salze aus Pyromellithsäure und 2-Phenyl-Δ2-imidazolin herzustellen. Danach führt die Umsetzung von einem Mol Pyromellithsäure mit einem Mol 2-Phenyl-Δ2-imidazolin in Lösungsmitteln wie Dimethylformamid, Dimethylacetamid, N-Methylpyrrolidon, aliphatischen Ketonen oder auch Alkoholen zu dem Monosalz der Pyromellithsäure, das wegen seiner Schwerlöslichkeit ausfällt.

Die Salze sind gemäss dem oben zitierten Schutzrecht Härter für Epoxidharze zur Herstellung von Überzügen mit besonders wertvollen Eigenschaften nach dem Pulverlackverfahren. So können bei Verwendung des Monosalzes der Pyromellithsäure mit 2-Phenyl-Δ2-imidazolin als Härter für Epoxidharze sehr matte Überzüge mit guten mechanischen Werten erhalten werden. Vor ihrer Anwendung werden die Pulverlackbestandteile unterhalb der Härtungstemperaturen innig vermischt, extrudiert und anschliessend gemahlen. Für die praktische Anwendung wird vorzugsweise eine Teilchengrösse <100 μm angestrebt, wobei das Teilchengrössenmaximum zwischen 30 und 50 μm liegen sollte. Dieses Epoxidpulverharz trägt man nun auf die zu lackierende Fläche auf und härtet es durch Erhitzen auf 160–240°C.

Nachteilig für dieses Verfahren ist die Notwendigkeit der Verwendung von organischen Lösungsmitteln bei der Herstellung der Salze. Man benötigt dazu sowohl zur Herstellung als auch zur Aufarbeitung geschlossene Apparaturen, hat mit giftigen und brennbaren Flüssigkeiten zu arbeiten und muss nach der Reaktion die Lösungsmittel durch Destillation reinigen, um sie wieder in den Verfahrenskreislauf zurückführen zu können.

Es hat deshalb nicht an Versuchen gefehlt, die organischen Lösungsmittel durch Wasser zu ersetzen. Dabei machte es sich nachteilig bemerkbar, dass das 2-Phenyl-Δ2-imidazolin, im Gegensatz zu den erwähnten organischen Lösungsmitteln, in Wasser schwer löslich ist.

Man geht normalerweise so vor, dass man z.B. Pyromellithsäure in Wasser vorlegt, derart, dass man zunächst bei 70–90°C eine klare Lösung oder zwischen 30–70°C eine Suspension der Säure in Wasser herstellt, wonach man die dem Monosalz entsprechende Menge 2-Phenyl-Δ2-imidazolin fest unter Rühren einträgt. Das nach etwa 2 Stunden Rühren zwischen 30 und 80°C gebildete Monosalz wird durch Filtration gewonnen. Bei der anwendungstechnischen Prüfung dieses auf diese Weise gewonnenen Monosalzes, z.B. aus Pyromellithsäure, stellte sich nun heraus, dass die mechanischen Eigenschaften des damit hergestellten Epoxidpulverlackes bei weitem nicht die hervorragenden Werte erreichen, die mit dem Monosalz nach DE-PS 2 324 696 erhalten worden waren. Darüber hinaus waren die physikalischen Werte schwankend, also nicht reproduzierbar.

Es stellte sich nun überraschend und in keiner Weise vorhersehbar heraus, dass das in Wasser hergestellte Monosalz nur durch Zerkleinerung auf Korngrössen <60 μ vor dem Mischen und Extrudieren mit dem ungehärteten Epoxidharz gemäss DE-PS 2 324 696 zu einem Härter wird, der in Epoxidpulverlacken zu Beschichtungen mit optimalen und reproduzierbaren physikalischen Eigenschaften führt. Der Effekt ist deshalb so überraschend, weil, wie oben ausgeführt, nach dem Mischen und Extrudieren des Härters mit dem ungehärteten Epoxidharz ohnehin ein Pulverisieren auf Korngrössen gleicher Grössenordnung zum Pulverlackharz durchgeführt wird. Diese Mahlung des Extrudates allein genügt also nicht zur Herstellung von gehärteten Pulverlacken optimaler Qualität.

Gegenstand der Erfindung ist daher ein Verfahren zur Herstellung von Epoxidpulverlacken mit Härtern aus in Wasser hergestellten Monosalzen von aromatischen Polycarbonsäuren mit mindestens drei Carboxylgruppen und 2-Phenyl-Δ2-imidazolin, wobei die Salze mit den Epoxidharzen vermischt, die Gemische unterhalb der Härtungstemperaturen extrudiert und anschliessend auf Teilchengrössen <100 μm gemahlen werden, dadurch gekennzeichnet, dass vor der Mischung mit dem Epoxidharz der Härter einer zusätzlichen Zerkleinerung auf Teilchengrössen <60 μm unterworfen wird.

Die Zerkleinerung des Härters auf Korngrössen unterhalb 60 μm kann auf beliebige Art, z.B. durch Mahlung, durchgeführt werden.

Beispiele:
A) Herstellung der Monosalze (Härter)
0,5 Mol Carbonsäure wurden mit 600 ml Wasser auf 60–80°C erhitzt und anschliessend mit 0,5 Mol 2-Phenyl-Δ2-imidazolin unter Rühren versetzt. Nach 2stündiger Nachreaktion bei gleicher Temperatur filtrierte man das ausgefallene Monosalz ab und trocknete es. Die Ausbeuten lagen zwischen 97 und 99% der Theorie. Die Reinheit der Salze wurde durch Röntgenstrukturanalysen bestätigt.

B) Zusammensetzung der Pulverlacke
54,5 Gew.% festes Epoxidharz, hergestellt aus 2,2-Bis-(4-hydroxyphenyl)-propan und Epichlorhydrin, mit dem Epoxid-Äquivalentgewicht von 900–1000 und einem Schmelzbereich von 90–100°C
5,0 Gew.% Monosalz als Härter
40,0 Gew.% Titandioxid in Pulverform
0,5 Gew.% handelsübliches Verlaufmittel auf Basis eines Polyacrylates.

C) Herstellung und Prüfung der Pulverlacke
Die Herstellung der Pulver und Beschichtungen sowie deren lacktechnische Prüfungen erfolgte gemäss den Beschreibungen aus DE-PS 2 324 696.

Die zur Salzbildung mit dem 2-Phenyl-Δ2-imidazolin verwendeten Carbonsäuren, die Kornverteilung der Härter und die Ergebnisse der lacktechnischen Prüfungen gehen aus der nachfolgenden Tabelle hervor. Die Bestimmungen der Kornverteilungsspektren der Härter vor und nach den Feinmahlungen wurden nach dem Micro-Videomat-Verfahren durchgeführt. Die Beispiele 1, 3, 5 und 7 sind jeweils Vergleichsversuche, die mit ungemahlenen Härtern durchgeführt wurden.

Tabelle

| Beispiel | 1 | 2 | 3 | 4 | 5 | 6 | 7 | 8 |
|---|---|---|---|---|---|---|---|---|
| Zur Salzbildung verwendete Carbonsäure: | Pyromellithsäure | | | | Trimellith-säure | | Trimesin-säure | |
| **Kornverteilungen der Härter in Vol.%:** | | | | | | | | |
| <4,76 µm | | 1,7 | | 3,2 | | 0,8 | | 1,1 |
| 4,76– 6,73 µm | | 3,8 | | 5,9 | | 2,6 | | 3,6 |
| 6,74– 9,51 µm | | 11,4 | 0,1 | 14,3 | | 8,7 | | 12,9 |
| 9,52– 13,45 µm | 0,1 | 22,1 | 0,3 | 20,6 | 0,2 | 21,6 | 0,1 | 19,7 |
| 13,46– 19,03 µm | 0,4 | 27,3 | 1,0 | 27,8 | 0,9 | 26,8 | 0,7 | 28,3 |
| 19,04– 26,91 µm | 0,8 | 14,3 | 1,8 | 19,4 | 1,8 | 22,0 | 1,2 | 24,8 |
| 26,92– 38,05 µm | 2,4 | 14,4 | 4,6 | 4,5 | 3,0 | 17,5 | 4,5 | 9,6 |
| 38,06– 53,82 µm | 5,4 | | 10,4 | 4,3 | 7,4 | | 7,2 | |
| 53,83– 76,11 µm | 12,7 | | 18,5 | | 18,5 | | 10,6 | |
| 76,12–107,03 µm | 21,9 | | 28,6 | | 27,6 | | 20,3 | |
| 107,04–152,22 µm | 24,1 | | 20,1 | | 30,2 | | 26,5 | |
| 152,23–215,27 µm | 14,8 | | 8,7 | | 5,3 | | 12,4 | |
| 215,28–304,44 µm | 16,9 | | 5,9 | | 5,1 | | 16,5 | |
| **Ergebnis der lacktechnischen Prüfungen:** | | | | | | | | |
| Schichtdicke [µm] | 60–70 | 65–70 | 55–65 | 60–70 | 60–65 | 60–70 | 50–60 | 50–60 |
| Glanzgrad n. Gardner 60° ⊰ [%] | 9 | 9 | 10 | 9 | 26 | 25 | 14 | 14 |
| Erichsentiefung (DIN 53156) [mm] | 1,0 | 7,5 | 1,4 | 6,2 | 1,2 | 8,2 | 0,8 | 5,6 |
| Dornbiegeversuch (DIN 53152) [mm] | 8 | <2 | 10 | <2 | 12 | <2 | 20 | <2 |
| **Kugelschlag n. Gardner** | | | | | | | | |
| direct impact [in lb] | 30 | 80 | 34 | 80 | 30 | >80 | 28 | 80 |
| reverse impact [in lb] | <10 | >80 | <10 | 60 | <10 | >80 | <10 | 40 |

## Patentanspruch

1. Verfahren zur Herstellung von Epoxidpulverlacken mit Härtern aus in Wasser hergestellten Monosalzen von aromatischen Polycarbonsäuren mit mindestens drei Carboxylgruppen und 2-Phenyl-Δ2-imidazolin, wobei die Salze mit den Epoxidharzen vermischt, die Gemische unterhalb der Härtungstemperaturen extrudiert und anschliessend auf Teilchengrössen <100 µm gemahlen werden, dadurch gekennzeichnet, dass vor der Mischung mit dem Epoxidharz der Härter einer zusätzlichen Zerkleinerung auf Teilchengrössen <60 µm unterworfen wird.

## Claim

Process for the preparation of epoxide powder lacquers with hardeners of monosalts, prepared in water, of aromatic polycarboxylic acids having at least three carboxyl groups and 2-phenyl-Δ2-imidazoline, the salts being mixed with the epoxide resins and the mixtures being extruded below the hardening temperatures and subsequently being ground to particle sizes of <100 µm, characterised in that before being mixed with the epoxide resin the hardener is subjected to an additional comminution to particle sizes of <60 µm.

## Revendication

Procédé pour la fabrication de vernis en poudre à base de résine époxyde avec des durcisseurs faits de monosels, fabriqués dans l'eau, d'acides polycarboxyliques aromatiques comportant au moins trois groupes carboxyle et de 2-phényl-Δ2-imidazoline, où les sels sont mélangés avec les résines époxydes, les mélanges sont extrudés à une température inférieure à celle du durcissement, et ensuite broyés à une grosseur de particules inférieure à 100 µ, procédé caractérisé en ce qu'avant de le mélanger avec la résine époxyde, on soumet le durcisseur à un broyage supplémentaire, à une grosseur de particules inférieure à 60 µ.